# EUROPÄISCHE PATENTSCHRIFT

(11) **EP 2 421 475 B1**
(45) Veröffentlichungstag und Bekanntmachung des Hinweises auf die Patenterteilung: **07.09.2016**
(21) Anmeldenummer: 10701327.8
(22) Anmeldetag: 26.01.2010
(51) Int. Cl.: A61F 2/36, A61F 2/48, A61F 2/30, A61F 2/28

(54) **ELEKTRISCHE HÜFTGELENKPROTHESE**
ELECTRIC HIP JOINT PROSTHESIS
PROTHÈSE DE HANCHE ÉLECTRIQUE

(30) Priorität: 03.02.2009 DE 102009007195
(43) Veröffentlichungstag der Anmeldung: 29.02.2012
(73) Patentinhaber: Neue Magnetodyn GmbH, 80333 München (DE)
(72) Erfinder: KRAUS, Werner, verstorben (DE); KRAUS, Stephanie, 80538 München (DE); STEPHAN, Heribert, 80689 München (DE); MILITZ, Matthias, 82418 Murnau (DE)
(74) Vertreter: Schumacher & Willsau
(86) Internationale Anmeldenummer: PCT/EP2010/000465
(87) Internationale Veröffentlichungsnummer: WO 2010/089044

(56) Entgegenhaltungen:
- EP-A2- 0 781 532
- DE-A1- 2 315 517
- DE-A1- 3 709 734
- DE-A1- 4 421 154
- US-A- 4 216 548

## Beschreibung

Die Erfindung betrifft eine Hüftgelenkprothese mit einem in einen Oberschenkelknochen einführbaren schaftförmigen Abschnitt und einer proximal angeordneten Endkappenanordnung, die einen Hohlraum der Hüftgelenkprothese verschließt.

Derartige Hüftgelenkprothesen sind auf dem Gebiet der Endoprothetik bekannt, beispielsweise aus der DE 295 06 036 U1. Die Druckschrift EP 0781532 A2 offenbart die Merkmale des Oberbegriffs des Anspruchs 1. Problematisch an derartigen unelastischen, gewebeverdrängenden Implantaten ist jedoch die Behinderung der biologischen Erholung, vor allem durch den Verlust von Blutgefäßen und Nerven. Außerdem leidet mit zunehmender Implantationsdauer die biomechanische Qualität der Stützstruktur durch den partiellen Entzug ihrer Funktion. Mit dem Verlust an biologischer Kontrolle aber wächst die Gefahr der Infektion durch resistente Bakterien (MRSA = Multiresistenter Staphylococcus Aureus). Es wurde gezeigt, dass diese die Oberfläche von Metallimplantaten in Form eines adhärenten Biofilmes besiedeln können und mit einer Schleimhülle aus Polysacchariden dem Angriff von Antibiotika widerstehen.

Diesen Problemen kann im Rahmen der orthopädischen Chirurgie durch die magnetisch induzierte Elektro-Osteotherapie begegnet werden. Beispiele hierfür sind die aus DE 10 2004 024 473 A1 und DE 199 28 449 A1 bekannt. Bei der Elektro-Osteotherapie werden in Endoprothesen oder Osteosythesemitteln elektrische Wechselpotentiale niedriger Frequenz dadurch induziert, dass ein betroffener Körperteil einem magnetischen Wechselfeld ausgesetzt wird. Seit langem wurde in zahlreichen klinischen Anwendungen der verfahrensgemäßen Technik bei chronisch therapieresistenten, meist infizierten Knochendefekten, Zysten und Tumormetastasen sowie in kliniknahen experimentellen Studien gezeigt, dass mit der Verwendung der Implantate als Quellen extrem niederfrequenter sinusförmiger elektrischer Wechselpotentiale in der dem Implantat anliegenden Knochenregion ein optimaler Heilungseffekt erzielt wird.

Die Technik der Übertragung funktioniert nach dem Transformatorprinzip: Die verletzte oder kranke Körperregion wird von einem extrem niederfrequenten sinusförmig verlaufenden Magnetfeld mit einer Frequenz von ca. 1 bis 100 Hz - vorzugsweise von 4 bis 20 Hz - und einer magnetischen Flussdichte von 0,5 bis 5 mT (5 bis 50 Gauß) durchflutet, das durch einen Funktionsstromgenerator in einer oder mehreren - primären - äußeren Stromspulen erzeugt wird, in die das mit dem Implantat versehene Körperteil eingebracht wird. Diese extrem niederfrequenten elektromagnetischen Felder durchdringen weitgehend verlustfrei das Gewebe, einschließlich eventuelle Kleidung und einen Gipsverband, sowie die unmagnetischen (austenitischen) Implantatmetalle. Im elektrischen Kontakt mit diesen wird eine - sekundäre - Spulenanordnung, der so genannte Übertrager, implantiert. Die in dem Übertrager induzierten Elektropotentiale werden so im Bereich der an das Implantat angrenzenden Gewebe zur Wirkung gebracht.

Mit dieser Technik der induktiven Übertragung therapeutisch wirksamer Elektropotentiale auf die Implantatbestandteile wird die Infektionsgefahr durch percutane Stromleitungen vermieden, und es können die Behandlungsparameter elektrische Spannung, Frequenz, Intensität, Signalform und die Behandlungszeit mit der indikationsspezifischen Programmierung eines Funktionsstrom-Generators des induzierenden Magnetfeldes bestimmt werden.

Der Erfindung liegt die Aufgabe zugrunde, ein orthopädisches Implantat in Form einer gattungsgemäßen Hüftgelenkprothese insbesondere im Hinblick auf ihre Handhabbarkeit und flexible Verwendbarkeit während der Operation, ihre biologische Wirkung, ihre therapeutische Wirksamkeit und ihre Wirtschaftlichkeit zu verbessern.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung baut auf der gattungsgemäßen Hüftgelenkprothese dadurch auf, dass die Endkappenanordnung eine Spulenanordnung trägt, welche mit einem ersten Pol mit einer ersten Elektrode und mit einem zweiten Pol mit einer zweiten Elektrode verbunden ist, dass die erste und die zweite Elektrode Oberflächen der Hüftgelenkprothese umfassen und dass die Spulenanordnung in einem Zustand, in dem die Endkappenanordnung den Hohlraum der Hüftgelenkprothese verschließt, zum größten Teil in dem Hohlraum angeordnet ist. Zu diesem Zweck weist die Endkappe vorzugsweise ein inneres Volumen auf, in dem sich eine um einen Eisenkern gewickelte Aufnehmerspule befindet. Diese ist nützlicherweise in die Endkappenanordnung eingeklebt. Die Enden der Spulenanordnung kommen dabei in geeigneten Kontakt mit elektrisch leitfähigen Oberflächen, welche Gewebeelektroden bilden. Auf der Grundlage der Erfindung erhält die Endkappe somit eine Doppelfunktion. Zum einen verhindert sie das Einwachsen von Bindegewebe und Knochen in die Prothese, das eine eventuell erwünschte spätere Explantation erschweren würde. Zum anderen beherbergt die Endkappe die Komponenten, die der Hüftgelenkprthese die elektrischen Eigenschaften vermittelt. Neben den erwähnten Vorteilen im Hinblick auf die Verwendung einer weitgehend, was die mechanischen Eigenschaften betrifft, gegenüber dem Stand der Technik unveränderten Hüftgelenkprothese ist weiterhin festzuhalten, dass der Chirurg während der Operation entscheiden kann, ob er die Prothese mit einer herkömmlichen Endkappe oder einer mit den elektrischen Komponenten ausgestatteten Endkappe verschließt. Außerdem sind die Bereitstellung und die Lagerhaltung magnetisch induzierbarer Endkappen wesentlich weniger aufwendig und damit kostengünstiger als die Bereitstellung von magnetisch induzierbaren Hüftgelenkprothesen mit den erforderlichen unterschiedlichen Abmessungen. Weitere biologische Vorteile sind: Die Infektionsgefahr wird durch eine verstärkte Durchblutung und eine Immunreaktion des stimulierten Gewebes gemindert, die Antibiotikaresistenz des multiresistenten Staphylococcus Aureus (MRSA) wird überwunden, und die Adhärenz von Bakterienfilmen auf der Oberfläche des Prothesenkörpers wird durch die magnetisch induzierte elektrische Aktivierung der Oberfläche vermieden. Im extrem niederfrequenten nichtthermischen Elektromagnetfeld, das den gesamten Hüftgelenkbereich durchflutet, wird an der Oberfläche der Gelenkprothese ein elektrisches Feld induziert, das sich in Versuchen, in vitro, als bakterizid wirksam erwiesen hat. Das gilt auch für die gefährliche Adhäsion von antibiotikaresistenten Keimen, z. B. in Form von Biofilmen, die durch das elektrodynamische Potential an der Oberfläche des Implantates vermieden wird. Damit eignet sich das gelenkprothetische Implantat besonders auch als sogenannter "Spacer", der bei infizierten künstlichen Hüftgelenken in einer zweizeitigen Operation zur Sterilisation und Regeneration des Knochens implantiert wird und nach dem Aufbau eines aseptischen, tragfähigen Knochens wieder gegen eine individuelle Gelenkprothese ausgetauscht werden kann.

Erfindungsgemäß ist ein proximal mit dem schaftförmigen Abschnitt verbundener, einen Hüftgelenkkopf tragender, zumindest einen Teil des Hohlraums aufweisender Halsabschnitt, vorgesehen, wobei der Hohlraum im Halsabschnitt die Endkappenanordnung vollständig oder nahezu vollständig aufnimmt und das Einführen eines Befestigungsmittels und eines Werkzeugs zum Befestigen des Halsabschnittes an dem schaftförmigen Abschnitt erlaubt, und vershließt die Endkappenanordnung den Hohlraum des Halsabschnittes proximal. Der Hohlraum des Halsabschnittes erhält hierdurch eine Doppelfunktion. Einerseits erlaubt er das Einführen eines Befestigungsmittels, insbesondere einer Schraube, und eines Werkzeugs zum Befestigen des Halsabschnittes an dem schaftförmigen Abschnitt, andererseits bietet er aber auch genügend Raum, um die Endkappenanordnung vollständig oder nahezu vollständig im Inneren des Implantates unterzubringen. Die Außenabmessungen des Implantats werden auf diese Weise gering gehalten, da durch die Elektrifizierung keine Vergrößerung des Implantats erforderlich ist.

Gemäß einer Variante ist vorgesehen, dass die erste Elektrode durch eine Kontaktoberfläche der Endkappenanordnung gebildet ist und dass die zweite Elektrode durch eine Kontaktoberfläche des schaftförmigen Abschnittes gebildet ist. Bei dieser und anderen Ausführungsformen kann die Endkappenanordnung ganz oder teilweise aus einer isolierenden Keramik bestehen, wobei ein elektrisch leitfähiger Oberflächenbereich durch das Aufbringen von elektrisch leitfähigem Material realisiert werden kann.

Ebenfalls ist es möglich, dass die erste Elektrode durch eine Kontaktoberfläche der Endkappenanordnung gebildet ist und dass die zweite Elektrode durch eine Kontaktoberfläche des Halsabschnittes gebildet ist. Will man aus irgendwelchen Gründen die Ausbreitung des elektrischen Feldes im Bereich des schaftförmigen Abschnittes vermeiden, so ist diese Anordnung der Elektroden besonders nützlich.

Es ist aber auch möglich und in vielen Situationen bevorzugt, dass die erste Elektrode durch eine Kontaktoberfläche der Endkappenanordnung gebildet ist und dass die zweite Elektrode durch eine Kontaktoberfläche des schaftförmigen Abschnittes und des Halsabschnittes gebildet ist. Steht der Halsabschnitt mit dem schaftförmigen Abschnitt in elektrischem Kontakt, so kann die Gesamtheit aus Schaftabschnitt und Halsabschnitt die eine Elektrode bilden, während die andere Elektrode durch eine Oberfläche der Endkappenanordnung gebildet ist.

Weiterhin kann vorgesehen sein, dass die erste Elektrode durch eine Kontaktoberfläche des Halsabschnittes gebildet ist und dass die zweite Elektrode durch eine Kontaktoberfläche des schaftförmigen Abschnittes gebildet ist. Die Endkappe ist somit nicht Teil der Elektrodenanordnung, sondern die Elektroden werden durch den Halsabschnitt und den schaftförmigen Abschnitt gebildet.

Gemäß einer besonders bevorzugten Ausführungsform der vorliegenden Erfindung ist vorgesehen, dass der Halsabschnitt mit dem schaftförmigen Abschnitt durch eine axial angeordnete elektrisch isolierende Schraube verbunden ist. So können die beiden Abschnitte des Implantats, also der schaftförmige Abschnitt und der Halsabschnitt voneinander isoliert bleiben und auf dieser Grundlage das Gegenelektrodenpaar bilden. Beispielsweise kann eine Schraube aus Zirkonoxid (ZrO₂) verwendet werden.

In einer Weiterbildung der Erfindung ist vorgesehen, dass in dem Hohlraum ein Medikamentenreservoir angeordnet ist, das ein Medikament enthält, und dass der Hohlraum mit einem Außenbereich der Hüftgelenkprothese über Öffnungen verbunden ist, die einen Übertritt des Medikaments aus dem Hohlraum in den Außenbereich gestatten.

Gemäß einer bevorzugten Ausführungsform ist vorgesehen, dass das Medikamentenreservoir ein Antibiotikum enthält.

Dabei erweist es sich als vorteilhaft, dass das Medikament fließfähig ist. Die erfindungsgemäße Vorrichtung ermöglicht auf diese Weise also eine lokale Antibiose durch die Aktivierung von dünnflüssigen Antibiotikalösungen auf der Oberfläche des Implantates und im unmittelbar angrenzenden Kochen- und Weichgewebe. Dies ist von besonderem Vorteil, da eine Antibiose durch die während der Therapie applizierten elektromagnetischen Felder aktiviert werden kann.

Der medikamentenbezogene Aspekt der Erfindung ist zum Beispiel so ausführbar, dass das Medikamentenreservoir zumindest teilweise durch einen druckpermeablen Behälter gebildet ist, welcher beim Verschließen des Hohlraums mit der Endkappenanordnung seinen Inhalt zumindest teilweise abgibt.

Beispielsweise ist es möglich, dass die Endkappenanordnung den druckpermeablen Behälter trägt.

In den Hohlraum des Halsabschnittes wird vor der Implantation ein mit einem Antibiotikum gefüllter ringförmiger Schlauch eingelegt, oder dieser wird durch die Endkappenanordnung getragen, beispielsweise, indem er auf ein konisch zulaufendes Ende der Endkappenanordnung gesteckt wird. Innerhalb des druckpermeablen Schlauches befindet sich eine dünnflüssige Lösung eines Antibiotikums. Die Wand des Schlauchringes besteht aus einem dünnen elastischen Kunststoff, der unter leichtem Druck beim Einsetzen der Endkappe, insbesondere also beim Einschrauben der Endkappe, für die Lösung durchlässig wird.

Ebenfalls ist es denkbar, dass das Medikamentenreservoir mit einer ansteuerbaren Einrichtung zusammenwirkt, wobei durch Ansteuerung der Einrichtung das Medikament zumindest teilweise abgegeben werden kann. Das Medikament, insbesondere das Antibiotikum, kann hierdurch gezielt zu beliebigen Zeitpunkten abgegeben werden; die Abgabe ist also nicht auf diejenige zum Zeitpunkt der Implantation beschränkt.

Beispielsweise kann vorgesehen sein, dass die ansteuerbare Einrichtung geeignet ist, einen körperextern erzeugten magnetischen Impuls unmittelbar oder mittelbar in mechanische Energie umzusetzen, welche die Abgabe des Medikaments bewirkt. Ein magnetischer Impuls kann zum Beispiel unter Mitwirkung permanentmagnetischer oder ferromagnetischer beweglicher Komponenten in mechanische Energie umgesetzt werden, so dass die Abgabe des Medikaments bewirkt wird. Neben dieser durch den magnetischen Impuls unmittelbar erzeugten mechanischen Energie ist es aber auch möglich, dass der magnetische Impuls durch eine Einrichtung erfasst und dann durch Schaltungstechnik in ein Signal zur Erzeugung mechanischer Energie eines Aktuators umgesetzt wird.

Ebenfalls ist es denkbar, dass die ansteuerbare Einrichtung einen elektrischen Energiespeicher, einen Empfänger, eine elektrische Steuerschaltung und einen von der elektrischen Steuerschaltung antreibbaren Aktuator aufweist, so dass durch ein körperextern erzeugtes Signal die Abgabe des Medikaments bewirkbar ist. Der antreibbare Aktuator kann insbesondere Bestandteil einer Medikamentenpumpe sein.

Von besonderem Vorteil ist, dass der elektrische Energiespeicher ein Akkumulator ist, der durch eine in der Spulenanordnung induzierte elektrische Spannung aufladbar ist. Hierdurch eignet sich die Variante mit elektrischem Energiespeicher auch für Langzeitanwendungen, da der Akkumulator jederzeit durch ein von außen angelegtes Magnetfeld, was ja im Rahmen der Therapie ohnehin eingesetzt wird, aufladbar ist und insofern die Energie für das Antreiben der Medikamentenpumpe zur Verfügung stellen kann.

Die Erfindung wird nun mit Bezug auf die begleitenden Zeichnungen anhand bevorzugter Ausführungsformen beispielhaft erläutert.

Es zeigen:
- Figur 1: eine perspektivische Explosionsdarstellung einer erfindungsgemäßen Hüftgelenkprothese;
- Figur 2: eine perspektivische Explosionsdarstellung einer Endkappenanordnung einer erfindungsgemäßen Hüftgelenkprothese und
- Figur 3: einen Axialschnitt durch eine Endkappenanordnung einer erfindungsgemäßen Hüftgelenkprothese.

Bei der nachfolgenden Beschreibung der Zeichnungen bezeichnen gleiche Bezugszeichen gleiche oder vergleichbare Komponenten.

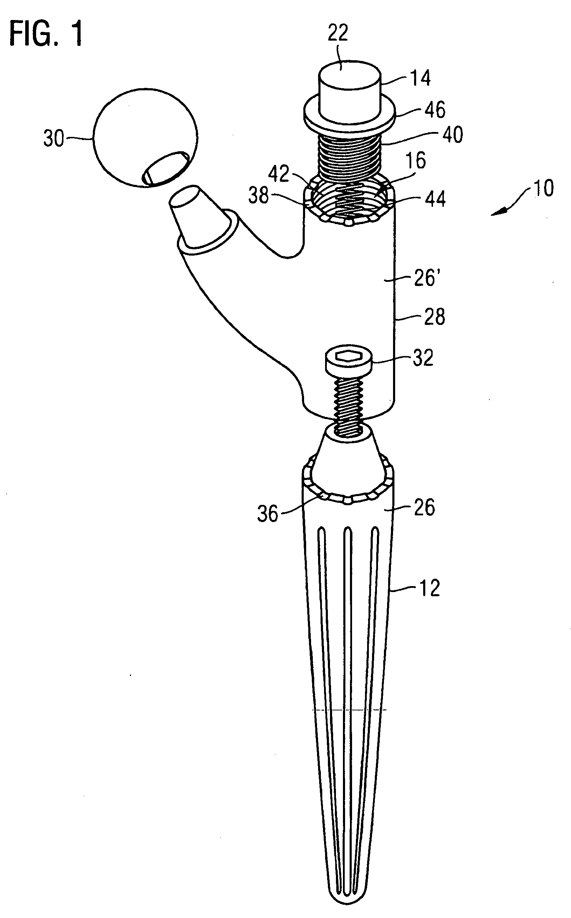

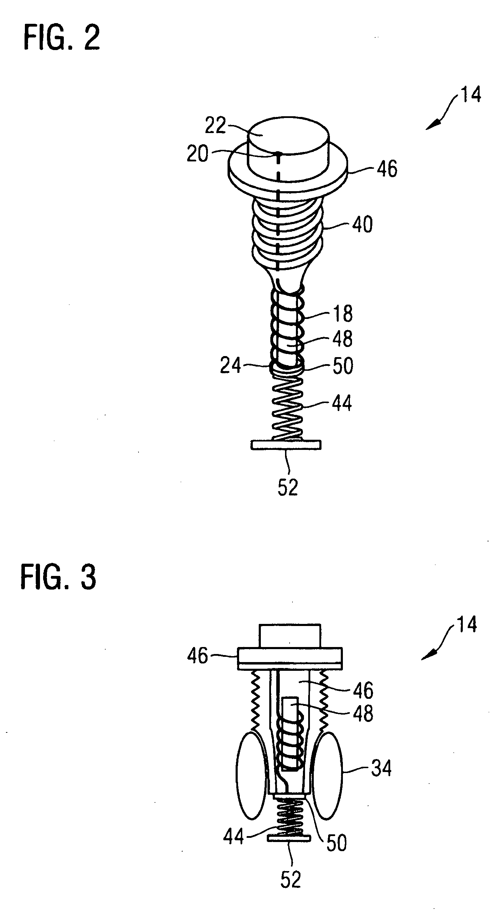

Figur 1 zeigt eine perspektivische Explosionsdarstellung einer erfindungsgemäßen Hüftgelenkprothese 10. Die Hüftgelenkprothese 10 hat einen schaftförmigen Abschnitt 12, einen Halsabschnitt 28, einen Hüftgelenkkopf 30 und eine Endkappenanordnung 14. Der Halsabschnitt 28 ist mit dem schaftförmigen Abschnitt 12 mittels einer Schraube 32 verbindbar. Die Endkappenanordnung 14 hat ein Außengewinde 40, mittels welchem diese in ein Innengewinde 42 des Halsabschnittes 28 einschraubbar ist. Innerhalb der Endkappenanordnung 14 sind elektrische Komponenten angeordnet, welche in Zusammenhang mit den Figuren 2 und 3 näher erläutert werden. Ein Pol dieser elektrischen Komponenten ist elektrisch mit einer Feder 44 verbunden, die von der Endkappenanordnung 14 getragen wird. Im zusammengebauten Zustand der Hüftgelenkprothese 10, das heißt bei einem mit dem schaftförmigen Abschnitt 12 durch die Schraube 32 verbundenen Halsabschnitt 28 und den Hohlraum 16 verschließender aufgeschraubter Endkappenanordnung 14 kontaktiert diese Feder 44 die axial angeordnete Schraube 32 mittelbar oder unmittelbar elektrisch, so dass der schaftförmige Abschnitt 12 und/oder der Halsabschnitt 28 das elektrische Potential der Feder 44 erhalten, so dass die leitfähigen Oberflächenabschnitte von schaftförmigem Abschnitt 12 und/oder Halsabschnitt zu Elektroden 26, 26' werden. Ein anderer Pol der elektrischen Anordnung steht beispielsweise mit einer Oberfläche der Endkappenanordnung 14 in Verbindung, so dass die Endkappe 14 die Gegenelektrode 22 zur vorstehend beschriebenen Elektrode 26, 26' bildet.

Figur 2 zeigt eine perspektivische Explosionsdarstellung einer Endkappenanordnung 14 einer erfindungsgemäßen Hüftgelenkprothese 10, und Figur 3 zeigt einen Axialschnitt durch eine Endkappenanordnung 14 einer erfindungsgemäßen Hüftgelenkprothese 10. Die Endkappenanordnung 14 hat einen Hohlraum 46. In diesen Hohlraum 46 ist eine auf einen Eisenkern 48 gewickelte Spulenanordnung 18 einschiebbar. Nach Einschieben der Anordnung in den Hohlraum 46 kann diese darin verklebt werden, beispielsweise mit Epoxidharzkleber, und der Hohlraum 46 kann distal mit einer Platte 50 verschlossen werden, vorzugsweise dadurch, dass die Platte 50 an dem sich in distale Richtung verjüngenden Gewindeteil angeklebt wird. Die metallische Platte 50 steht mit einem Pol 24 der Spulenanordnung 18 in elektrischem Kontakt. Der andere Pol 20 der Spulenanordnung 18 steht mit einer elektrisch leitenden Oberfläche 22 der Endkappenanordnung 14 in Verbindung. An die Platte 50 schließt in distale Richtung die bereits im Zusammenhang mit Figur 1 erwähnte elektrisch leitfähige Feder 44 an, an der wiederum eine elektrich leitfähige Platte 52 distal befestigt ist. Is die Endkappenanordnung 14 in die Hüftgelenkprothese eingesetzt, so schließt die Platte 52 einen elektrischen Kontakt mit der Schraube 32 (siehe Figur 1). Auf dieser Grundlage können verschiedene Konfigurationen von Oberflächenelektroden realisiert werden. Beispielsweise kann der das Gewinde 40 tragende Körper der Endkappenanordnung 14 elektrisch isolierend ausgeführt sein, vorzugsweise aus einer Aluminiumoxid- oder Zirkonoxid-Keramik (Al₂O₃ bzw. ZrO₂). Ein Teil der Oberfläche der Endkappenanordnung wird dann in einen elektrisch leitfähigen Zustand überführt, beispielsweise durch Aufsputtern oder Bedampfen mit elektrisch leitfähigem Material. Indem die metallische Platte 52 mit der Schraube 32 in Verbindung steht und diese wiederum sowohl den Halsabschnitt 28 als auch den schaftförmigen Abschnitt 12 - beide aus elektrisch leitfähigem Metall gefertigt - kontaktiert, bilden der Halsabschnitt 28 und der schaftförmige Abschnitt 12 gemeinsam die Gegenelektrode zu der elektrisch leitfähigen Oberfläche der Endkappenanordnung 14. Ebenfalls ist es denkbar, die Schraube 32 isolierend auszustatten und auch ansonsten dafür zu sorgen, dass kein elektrischer Kontakt zwischen dem Halsabschnitt 28 und dem schaftförmigen Abschnitt 12 besteht. Dann können durch elektrische Kontaktierung der Halsabschnitt 28 oder Teile des Halsabschnittes 28 alleine die Gegenelektrode zur Endkappenanordnung 14 bilden. Ebenfalls kann ein elektrischer Kontakt zwischen dem distalen Pol der Spulenanordnung und dem Halsabschnitt 28 vermieden werden, während ein elektrischer Kontakt zwischen diesem Pol 24 und dem schaftförmigen Abschnitt hergestellt wird. Dann bilden die elektrisch leitfähige Oberfläche der Endkappenanordnung 14 und der schaftförmige Abschnitt 12 Gegenelektroden ohne Einbeziehung des Halsabschnittes 28. Mit einer weiteren Ausführungsform ist es auch möglich, dass die Endkappenanordnung 14 nicht als Elektrode dient. Dies kann so realisiert sein, dass die Endkappenanordnung 14 an ihrer Oberfläche vollständig isolierend ist und der erste Pol 20 der Spulenanordnung 18 beim Einschrauben der Endkappenanordnung 14 in den Halsabschnitt 28 diesen kontaktiert, etwa durch eine Quetschkontaktierung mittels des Kragens 46 der Endkappenanordnung.

Figur 3 zeigt eine nützliche Ausführungsform einer Endkappenanordnung 14 mit einer weiteren Funktionalität. Die Endkappenanordnung 14 trägt ein Medikamentenreservoir 34. Dieses Medikamentenreservoir ist insbesondere zur lokalen Antibiose vorgesehen. Die äußere Oberfläche des Medikamentenreservoirs 34 ist als druckpermeable Wand ausgestattet, so dass eine flüssige Medikamentenlösung austreten kann, wenn die Endkappenanordnung 14 in den Hohlraum 16 (siehe Figur 1) eingeschraubt wird, da dann ein Zusammenpressen des Medikamentenreservoirs 34 erfolgt. Aus dem Medikamentenreservoir 34 austretende Flüssigkeit kann dann durch die Öffnungen 36, 38 aus dem Hohlraum 16 in den Außenraum der Hüftgelenkprothese 10 austreten. Da es auch nützlich sein kann, die Medikamentenlösung nicht bereits beim Einschrauben der Endkappenanordnung 14 in den Halsabschnitt 28 austreten zu lassen, kann vorgesehen sein, dass in dem Hohlraum 16 des Halsabschnittes 28 eine Medikamentenpumpe vorgesehen ist, die von der Spulenanordnung 18 mittelbar oder unmittelbar mit elektrischer Energie versorgt wird. Beispielsweise kann die Spulenanordnung 18 einen Akkumulator aufladen, der dann jederzeit die erforderliche elektrische Energie für den Betrieb der Medikamentenpumpe zur Verfügung stellt. Der Betrieb der Medikamentenpumpe kann durch eine herkömmliche Sende-Empfangseinrichtung gesteuert werden. Ebenfalls ist es denkbar, die für das zeitlich gesteuerte Austreten erforderliche mechanische Energie durch eine Komponente freizusetzen, die auf einen magnetischen Impuls reagiert.

### Bezugszeichenliste

- 10: Hüftgelenkprothese
- 12: schaftförmiger Abschnitt
- 14: Endkappenanordnung
- 16: Hohlraum
- 18: Spulenanordnung
- 20: erster Pol
- 22: erste Elektrode
- 24: zweiter Pol
- 26: zweite Elektrode
- 28: Halsabschnitt
- 30: Hüftgelenkkopf
- 32: Befestigungsmittel; elektrisch isolierende Schraube
- 34: Medikamentenreservoir; druckpermeabler Behälter
- 36: Öffnung
- 38: Öffnung
- 40: Außengewinde
- 42: Innengewinde
- 44: elektrisch leitfähige Feder
- 46: Hohlraum
- 48: Eisenkern
- 50: Platte 52 Platte

## Patentansprüche

1. Hüftgelenkprothese (10) mit
- einem in einen Oberschenkelknochen einführbaren schaftförmigen Abschnitt (12) und
- einer proximal angeordneten Endkappenanordnung (14), die einen Hohlraum (16) der Hüftgelenkprothese (10) verschließt,
- wobei die Endkappenanordnung (14) eine Spulenanordnung (18) trägt, welche mit einem ersten Pol (20) mit einer ersten Elektrode (22) und mit einem zweiten Pol (24) mit einer zweiten Elektrode (26) verbunden ist,
- wobei die erste und die zweite Elektrode (22, 26) Oberflächen der Hüftgelenkprothese (10) umfassen,
- wobei die Spulenanordnung (18) in einem Zustand, in dem die Endkappenanordnung (14) den Hohlraum (16) der Hüftgelenkprothese (10) verschließt, zum größten Teil in dem Hohlraum (16) angeordnet ist,
**dadurch gekennzeichnet, dass**
- ein proximal mit dem schaftförmigen Abschnitt (12) verbundener, einen Hüftgelenkkopf (30) tragender, zumindest einen Teil des Hohlraums aufweisender Halsabschnitt (28), vorgesehen ist, wobei der Hohlraum (16) im Halsabschnitt (28) die Endkappenanordnung (14) vollständig oder nahezu vollständig aufnimmt und das Einführen eines Befestigungsmittels (32) und eines Werkzeugs zum Befestigen des Halsabschnittes (28) an dem schaftförmigen Abschnitt (12) erlaubt, und dass
- die Endkappenanordnung (14) den Hohlraum (16) des Halsabschnittes (28) proximal verschließt.

2. Hüftgelenkprothese (10) nach Anspruch 1, **dadurch gekennzeichnet,**
- **dass** die erste Elektrode (22) durch eine Kontaktoberfläche der Endkappenanordnung (14) gebildet ist und
- **dass** die zweite Elektrode (26) durch eine Kontaktoberfläche des schaftförmigen Abschnittes (12) gebildet ist.

3. Hüftgelenkprothese (10) nach Anspruch 2, **dadurch gekennzeichnet,**
- **dass** die erste Elektrode (22) durch eine Kontaktoberfläche der Endkappenanordnung (14) gebildet ist und
- **dass** die zweite Elektrode (26') durch eine Kontaktoberfläche des Halsabschnittes (28) gebildet ist.

4. Hüftgelenkprothese (10) nach Anspruch 1, **dadurch gekennzeichnet,**
- **dass** die erste Elektrode (22) durch eine Kontaktoberfläche der Endkappenanordnung gebildet ist und
- **dass** die zweite Elektrode (26, 26') durch eine Kontaktoberfläche des schaftförmigen Abschnittes und des Halsabschnittes gebildet ist.

5. Hüftgelenkprothese (10) nach Anspruch 1, **dadurch gekennzeichnet,**
- **dass** die erste Elektrode (22) durch eine Kontaktoberfläche des Halsabschnittes (28) gebildet ist und
- **dass** die zweite Elektrode (26, 26') durch eine Kontaktoberfläche des schaftförmigen Abschnittes (12) gebildet ist.

6. Hüftgelenkprothese (10) nach Anspruch 5, **dadurch gekennzeichnet, dass** der Halsabschnitt (28) mit dem schaftförmigen Abschnitt (12) durch eine axial angeordnete elektrisch isolierende Schraube (32) verbunden ist.

7. Hüftgelenkprothese (10) nach einem der vorhergehenden Ansprüche, **dadurch gekennzeichnet,**
- **dass** in dem Hohlraum (16) ein Medikamentenreservoir (34) angeordnet ist, das ein Medikament enthält, und
- **dass** der Hohlraum (16) mit einem Außenbereich der Hüftgelenkprothese (10) über Öffnungen (36, 38) verbunden ist, die einen Übertritt des Medikaments aus dem Hohlraum (16) in den Außenbereich gestatten.

8. Hüftgelenkprothese (10) nach Anspruch 7, **dadurch gekennzeichnet, dass** das Medikamentenreservoir (34) ein Antibiotikum enthält.

9. Hüftgelenkprothese (10) nach Anspruch 7 oder 8, **dadurch gekennzeichnet, dass** das Medikament fließfähig ist.

10. Hüftgelenkprothese (10) nach Anspruch 7 oder 8, **dadurch gekennzeichnet, dass** das Medikamentenreservoir (34) zumindest teilweise durch einen druckpermeablen Behälter gebildet ist, welcher beim Verschließen des Hohlraums (16) mit der Endkappenanordnung (14) seinen Inhalt zumindest teilweise abgibt.

11. Hüftgelenkprothese (10) nach Anspruch 10, **dadurch gekennzeichnet, dass** die Endkappenanordnung (14) den druckpermeablen Behälter (34) trägt.

12. Hüftgelenkprothese (10) nach einem der Ansprüche 7 bis 11, **dadurch gekennzeichnet, dass** das Medikamentenreservoir (34) mit einer ansteuerbaren Einrichtung zusammenwirkt, wobei durch Ansteuerung der Einrichtung das Medikament zumindest teilweise abgegeben werden kann.

13. Hüftgelenkprothese (10) nach Anspruch 12, **dadurch gekennzeichnet, dass** die ansteuerbare Einrichtung geeignet ist, einen körperextern erzeugten magnetischen Impuls unmittelbar oder mittelbar in mechanische Energie umzusetzen, welche die Abgabe des Medikaments bewirkt.

14. Hüftgelenkprothese (10) nach Anspruch 12 oder 13, **dadurch gekennzeichnet, dass** die ansteuerbare Einrichtung einen elektrischen Energiespeicher, einen Empfänger, eine elektrische Steuerschaltung und einen von der elektrischen Steuerschaltung antreibbaren Aktuator aufweist, so dass durch ein körperextern erzeugtes Signal die Abgabe des Medikaments-bewirkbar ist.

15. Hüftgelenkprothese (10) nach Anspruch 14, **dadurch gekennzeichnet, dass** der elektrische Energiespeicher ein Akkumulator ist, der durch eine in der Spulenanordnung induzierte elektrische Spannung aufladbar ist.

## Claims

1. Hip joint prosthesis (10) with
- a stem-shaped portion (12) insertable into a femur, and
- a proximally arranged end cap assembly (14), which closes a hollow space (16) of the hip joint prosthesis (10),
- wherein the end cap assembly (14) carries a coil assembly (18), which is connected to a first pole (20) with a first electrode (22) and to a second pole (24) with a second electrode (26),
- wherein the first and the second electrode (22, 26) comprise surfaces of the hip joint prosthesis (10),
- wherein the coil assembly (18) is arranged for the most part in the hollow space (16) in a state in which the end cap assembly (14) closes the hollow space (16) of the hip joint prosthesis (10),
**characterized in that**
- a neck portion (28) connected proximally to the stem-shaped portion (12), carrying a femoral head (30) and comprising at least part of the hollow space (16) is provided, wherein the hollow space (16) in the neck portion (28) receives the end cap assembly (14) completely or almost completely and permits the insertion of a fastening means (32) and of a tool for fastening the neck portion (28) to the stem-shaped portion (12), and
- the end cap assembly (14) proximally closes the hollow space (16) of the neck portion (28).

2. Hip joint prosthesis (10) according to Claim 1, **characterized in that**
- the first electrode (22) is formed by a contact surface of the end cap assembly (14), and
- the second electrode (26) is formed by a contact surface of the stem-shaped portion (12).

3. Hip joint prosthesis (10) according to Claim 2, **characterized in that**
- the first electrode (22) is formed by a contact surface of the end cap assembly (14), and
- the second electrode (26') is formed by a contact surface of the neck portion (28).

4. Hip joint prosthesis (10) according to Claim 1, **characterized in that**
- the first electrode (22) is formed by a contact surface of the end cap assembly, and
- the second electrode (26, 26') is formed by a contact surface of the stem-shaped portion and of the neck portion.

5. Hip joint prosthesis (10) according to Claim 1, **characterized in that**
- the first electrode (22) is formed by a contact surface of the neck portion (28), and
- the second electrode (26, 26') is formed by a contact surface of the stem-shaped portion (12).

6. Hip joint prosthesis (10) according to Claim 5, **characterized in that** the neck portion (28) is connected to the stem-shaped portion (12) by an axially arranged, electrically insulating screw (32).

7. Hip joint prosthesis (10) according to one of the preceding claims, **characterized in that**
- a medicament reservoir (34) containing a medicament is arranged in the hollow space (16), and
- the hollow space (16) is connected to an outer area of the hip joint prosthesis (10) via openings (36, 38), which allow the medicament to cross from the hollow space (16) to the outer area.

8. Hip joint prosthesis (10) according to Claim 7, **characterized in that** the medicament reservoir (34) contains an antibiotic.

9. Hip joint prosthesis (10) according to Claim 7 or 8, **characterized in that** the medicament is free-flowing.

10. Hip joint prosthesis (10) according to Claim 7 or 8, **characterized in that** the medicament reservoir (34) is formed at least partially by a pressure-permeable container, which at least partially discharges its content when the hollow space (16) is closed by the end cap assembly (14).

11. Hip joint prosthesis (10) according to Claim 10, **characterized in that** the end cap assembly (14) carries the pressure-permeable container (34).

12. Hip joint prosthesis (10) according to one of Claims 7 to 11, **characterized in that** the medicament reservoir (34) cooperates with a actuatable device, wherein the medicament can be at least partially discharged by actuation of the device.

13. Hip joint prosthesis (10) according to Claim 12, **characterized in that** the actuatable device is suitable for directly or indirectly converting a magnetic impulse, generated outside of the body, into mechanical energy that effects the discharge of the medicament.

14. Hip joint prosthesis (10) according to Claim 12 or 13, **characterized in that** the actuatable device has an electrical energy store, a receiver, an electrical control circuit, and an actuator that can be driven by the electrical control circuit, such that the discharge of the medicament can be effected by a signal generated outside the body.

15. Hip joint prosthesis (10) according to Claim 14, **characterized in that** the electrical energy store is an accumulator, which can be charged by an electrical voltage induced in the coil assembly.

## Revendications

1. Prothèse de hanche (10) avec
- un tronçon (12) en forme de tige susceptible d'être introduit dans un fémur et
- un dispositif de capuchon de terminaison (14), placé du côté proximal qui ferme une cavité (16) de la prothèse de hanche (10),
- le dispositif de capuchon de terminaison (14) portant un dispositif de bobine (18) qui par un premier pôle (20) est relié avec une première électrode (22) et par un deuxième pôle (24) est relié avec une deuxième électrode (26),
- la première et la deuxième électrodes (22, 26) englobant des surfaces de la prothèse de hanche (10),
- dans une position dans laquelle le dispositif de capuchon de terminaison (14) ferme la cavité (16) de la prothèse de hanche (10), le dispositif de bobine (18) étant placé en majeure partie dans la cavité (16),
**caractérisée en ce**
**qu'**il est prévu un tronçon de col (28) relié côté proximal avec le tronçon (12) en forme de tige, portant une tête fémorale (30), comportant au moins une partie de la cavité, la cavité (16) dans le tronçon de col (28) réceptionnant totalement ou presque totalement le dispositif de capuchon de terminaison (14) et permettant l'introduction d'un moyen de fixation (32) et d'un outil pour la fixation du tronçon de col (28) sur le tronçon (12) en forme de tige et en ce que
- le dispositif de capuchon de terminaison (14) ferme la cavité (16) du tronçon de col (28) du côté proximal.

2. Prothèse de hanche (10) selon la revendication 1, **caractérisée en ce que**
- la première électrode (22) est formée par une surface de contact du dispositif de capuchon de terminaison (14) et
- **en ce que** la deuxième électrode (26) est formée par une surface de contact du tronçon (12) en forme de tige.

3. Prothèse de hanche (10) selon la revendication 2, **caractérisée en ce que**
- la première électrode (22) est formée par une surface de contact du dispositif de capuchon de terminaison (14) et
- **en ce que** la deuxième électrode (26') est formée par une surface de contact du tronçon de col (28).

4. Prothèse de hanche (10) selon la revendication 1,
**caractérisée en ce que**
- la première électrode (22) est formée par une surface de contact du dispositif de capuchon de terminaison et
- **en ce que** la deuxième électrode (26, 26') est formée par une surface de contact du tronçon en forme de tige et du tronçon de col.

5. Prothèse de hanche (10) selon la revendication 1, **caractérisée en ce que**
- la première électrode (22) est formée par une surface de contact du tronçon de col (28) et
- **en ce que** la deuxième électrode (26, 26') est formée par une surface de contact du tronçon (12) en forme de tige.

6. Prothèse de hanche (10) selon la revendication 5, **caractérisée en ce que** le tronçon de col (28) est relié avec le tronçon (12) en forme de tige par une vis isolante électrique (32) placée en direction axiale.

7. Prothèse de hanche (10) selon l'une quelconque des revendications précédentes, **caractérisée en ce que**
- dans la cavité (16) est placé un réservoir à médicament (34) qui contient un médicament et
- **en ce que** la cavité (16) est reliée avec une région extérieure de la prothèse de hanche (10) par l'intermédiaire d'orifices (36, 38) qui admettent un passage du médicament de la cavité (16) dans la région extérieure.

8. Prothèse de hanche (10) selon la revendication 7, **caractérisée en ce que** le réservoir à médicament (34) contient un antibiotique.

9. Prothèse de hanche (10) selon la revendication 7 ou la revendication 8, **caractérisée en ce que** le médicament est fluide.

10. Prothèse de hanche (10) selon la revendication 7 ou la revendication 8, **caractérisée en ce que** le réservoir à médicament (34) est formé au moins en partie par un récipient perméable à la pression, lequel lors de la fermeture de la cavité (16) avec le dispositif de capuchon de terminaison (14) distribue au moins en partie son contenu.

11. Prothèse de hanche (10) selon la revendication 10, **caractérisée en ce que** le dispositif de capuchon de terminaison (14) porte le récipient (34) perméable à la pression.

12. Prothèse de hanche (10) selon l'une quelconque des revendications 7 à 11, **caractérisée en ce que** le réservoir à médicament (34) interagit avec un système amorçable, le médicament pouvant, par amorçage du système, être distribué au moins en partie.

13. Prothèse de hanche (10) selon la revendication 12, **caractérisée en ce que** le système amorçable est apte à transformer directement ou indirectement une impulsion magnétique générée à l'extérieur du corps en une énergie mécanique, laquelle provoque la distribution du médicament.

14. Prothèse de hanche (10) selon la revendication 12 ou la revendication 13, **caractérisée en ce que** le système amorçable comporte un accumulateur d'énergie électrique, un récepteur, un circuit de commande électrique et un actionneur susceptible d'être activé par le circuit de commande électrique, de sorte que par un signal généré en externe du corps, la distribution du médicament puisse être provoquée.

15. Prothèse de hanche (10) selon la revendication 14, **caractérisée en ce que** l'accumulateur d'énergie électrique est un accumulateur qui peut être chargé par une tension électrique induite dans le dispositif de bobine.
